# EUROPEAN PATENT SPECIFICATION

(11) **EP 3 799 841 B1**
(45) Date of publication and mention of the grant of the patent: **18.11.2026**
(21) Application number: 20196635.5
(22) Date of filing: 17.09.2020
(51) Int. Cl.: A61F 2/46, A61B 17/92

(54) **MEDICAL IMPLANT EXTRACTOR**
EXTRAKTOR FÜR MEDIZINISCHE IMPLANTATE
EXTRACTEUR IMPLANTS MÉDICAUX

(30) Priority: 01.10.2019 US 201962908807 P; 17.01.2020 US 202016746599
(43) Date of publication of application: 07.04.2021
(73) Proprietor: Shukla Medical, St. Petersburg, FL 33709 (US)
(72) Inventor: SWEITZER, Zachary Robert, Keyport, NJ 07735 (US)
(74) Representative: Cameron Intellectual Property Ltd

(56) References cited:
- WO-A1-2019/009891
- CN-U- 202 821 729
- US-A1- 2011 184 411
- US-A1- 2013 211 404
- US-A1- 2013 261 626

## Description

### BACKGROUND OF THE DISCLOSURE

The present disclosure relates generally to a medical device and, more specifically, to a medical implant extractor for extracting a medical implant from bone. The closest prior art is document US 2013/261626 A1, which defines the preamble of claim 1.

Medical implant extraction devices for extracting implants from bone assume various forms and modes of operation. Some include devices that clamp an implant prior to implant extraction. Such devices involve moving clamp parts that oftentimes require two-handed operation: one hand to operate the movable clamp and the other to pull the clamped implant from the bone in which it is embedded. Other devices are constructed as chisels that are used to pry the implant from the bone. During implant extraction, such devices require considerable radial clearance to enable the device to be moved about the circumference of the implant during the prying process, and as such may interfere with surrounding bone or other bodily tissue as they are moved about the implant.

US 2011/184411 A1 discloses a surgical tool for pectus bar extraction.

US 2013/211404 A1 discloses a another surgical tool for pectus bar extraction.

### BRIEF SUMMARY OF THE DISCLOSURE

A medical implant extractor according to the invention is defined in claim 1.

Optionally, the shank includes a first curved segment and a second curved segment. Optionally, the second axial segment is spaced axially from the first axial segment.

Optionally, the second axial segment is laterally spaced from the first axial segment. Optionally, the first axial segment is spaced laterally from the second axial segment about 0 to 150 mm.

Optionally, the tip defines a most distal end of the medical implant extractor. Optionally, the tip includes a recess about its midportion. Optionally, the tip extends past a most lateral end of the handle about 0 to 150 mm.

Optionally, the head includes a tapered end defining the tip. Optionally, the head includes a distally facing end that is substantially curved. Optionally, the head includes a distally facing end that includes a plurality of planar segments.

Optionally, the strike plate is positioned between planes defined by the lateral most ends of the handle. Optionally, the medical implant extractor further comprises a second strike plate about a proximally facing end of the handle. Optionally, the medical implant extractor further comprises a third strike plate about a distally facing end of the handle.

The head is of compact construction. Furthermore, optionally, the first and second longitudinal axes of the first and second axial segments are laterally spaced to position the head such that the tip can be inserted beneath a portion of an implant while the remainder of the head occupies minimal radial space from the implant. So constructed, effective implant extraction force may be exerted on the head by striking the strike plate with a striking tool in a substantially radial direction while minimizing the radial space within which the striking tool is swung.

Other features and advantages of the subject disclosure will be apparent from the following more detail description of the examples.

### BRIEF DESCRIPTION OF THE SEVERAL VIEWS OF THE DRAWINGS

The foregoing summary, as well as the following detailed description of the examples of the subject disclosure, will be better understood when read in conjunction with the appended drawings. For the purpose of illustrating the present disclosure, there are shown in the drawings examples of the device. It should be understood, however, that the subject application is not limited to the precise arrangements and instrumentalities shown.
FIG. 1 is a side elevation view of a medical implant extractor;
FIG. 2 is a rear perspective view of the medical implant extractor of FIG. 1;
FIG. 3 is a rear perspective view of the medical implant extractor of FIG. 1 engaged with a medical implant embedded in an upper region of a humerus bone; and
FIG. 4 is a side view of the medical implant extractor of FIG. 1 engaged with a medical implant embedded in an upper region of a humerus bone.

### DETAILED DESCRIPTION OF THE DISCLOSURE

Reference will now be made in detail to the various examples of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject application in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art.

Throughout the subject application, various aspects thereof can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the examples of the subject disclosure may be combined in any suitable manner in one or more examples. One skilled in the relevant art will recognize, in light of the description herein, that the subject disclosure can be practiced without one or more of the specific features or advantages of a particular example. In other instances, additional features and advantages may be recognized in certain examples that may not be present in all exemplary examples of the present disclosure.

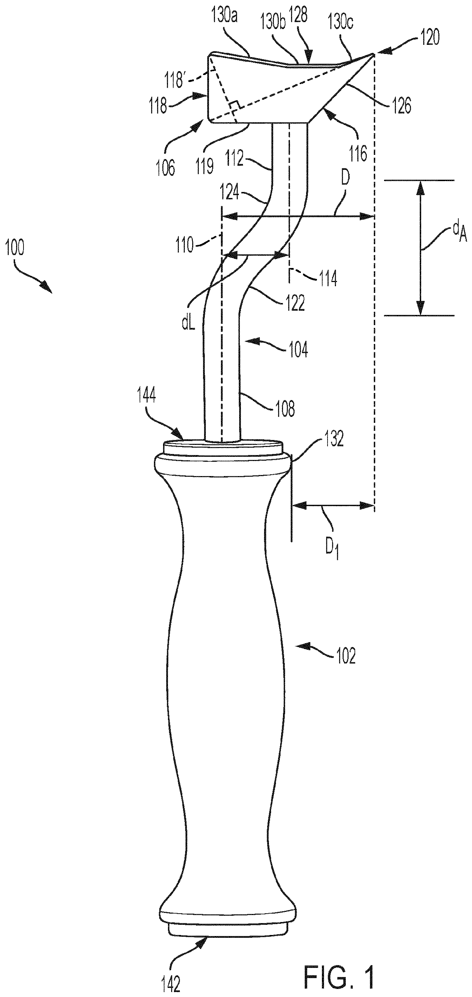

Referring now to the drawings, FIG. 1 illustrates a medical implant extractor 100. The medical implant extractor 100 includes a handle 102, a shank 104 extending from the handle, and a head 106 connected to a distal end of the shank.

The handle 102 is constructed to be ergonomically shaped to provide a secure grip by a user, e.g., a surgeon. The handle may further be formed from or surrounded by grip enhancing material to promote a firm grip by a user as well as be sterilizable.

The shank includes a first axial segment 108 having a first longitudinal axis 110, and a second axial segment 112 having a second longitudinal axis 114 spaced from the first longitudinal axis. The second axial segment 112 of the shank 104 is spaced axially from the first axial segment 108. According to an example, the first axial segment 108 is spaced axially from the second axial segment 112 a distance "d_{A}" of about 0 to 200 mm. In addition, the second axial segment 112 is laterally spaced from the first axial segment 108. According to an example, the first axial segment 108 is spaced laterally from the second axial segment 112 a distance "d_{L}" of about 0 to 150 mm.

In addition to the first and second axial segments 108, 112, the shank 104 includes a first curved segment 122 and a second curved segment 124 that joins the first axial segment to the second axial segment. Together, the first curved segment 122 and second curved segment form a generally S-shaped portion. Moreover, the generally S-shaped portion can alternatively be made from non-curved segments such as a plurality of linear segments, e.g., forming a substantially "Z" shaped portion between the first and second axial segments 108, 112.

The head 106 is connected to a distal end of the second axial segment 112. The head includes a main body 116, a strike plate 118 facing substantially parallel to the longitudinal axis of one of the first and second longitudinal axes 110, 114 of the first and second axial segments 108, 112, and a tip 120 extending distally and outwardly from the main body. According to another example, the strike plate, identified by reference numeral 118' can be substantially perpendicular to the tip 120. In addition to the strike plate 118, the medical implant extractor can further comprise a second strike plate 142 about a proximally facing end of the handle 102, and a third strike plate 144 about a distally facing end of the handle. The functions of the strike plate 118, the second strike plate 142 and the third strike plate 144 are described below.

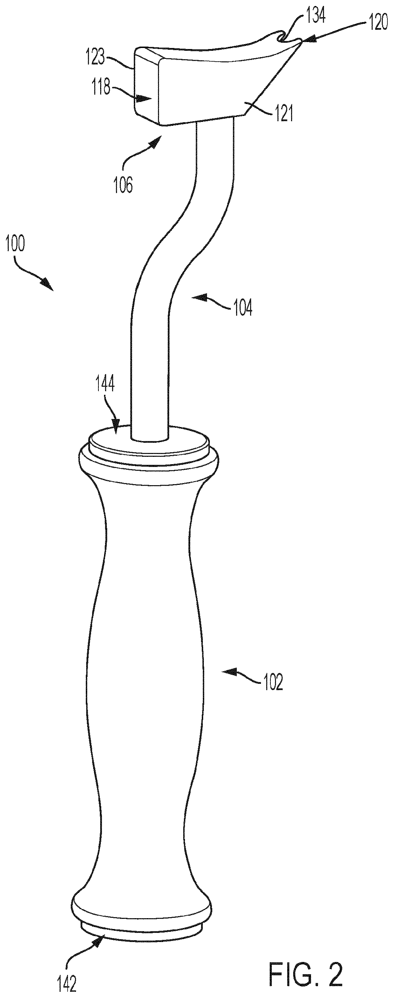

The head 106 is of compact construction; that is, its length from the strike plate 118 to the tip 120 is about 45 mm, its height from the tip to a bottom 119 of the head is about 18 mm, and its width between lateral faces 121, 123 of the head (FIG. 2) is about 13 mm. The head further includes a tapered end 126 defining the tip 120 and a distally facing end 128 that is substantially curved. According to an example, the substantially curved distally facing end 128 includes a plurality of planar segments 130a, 130b, 130c defining the substantially curved distally facing end.

The shank 104 and head 106 may be formed from any substantially rigid material including, without limitation, metal such as stainless steel, composite, or reinforced plastic, which is capable of withstanding the impact forces experienced when extracting an implant, e.g., a glenosphere implant, a glenoid implant, a knee implant, a hip implant, an elbow implant or other implant from bone.

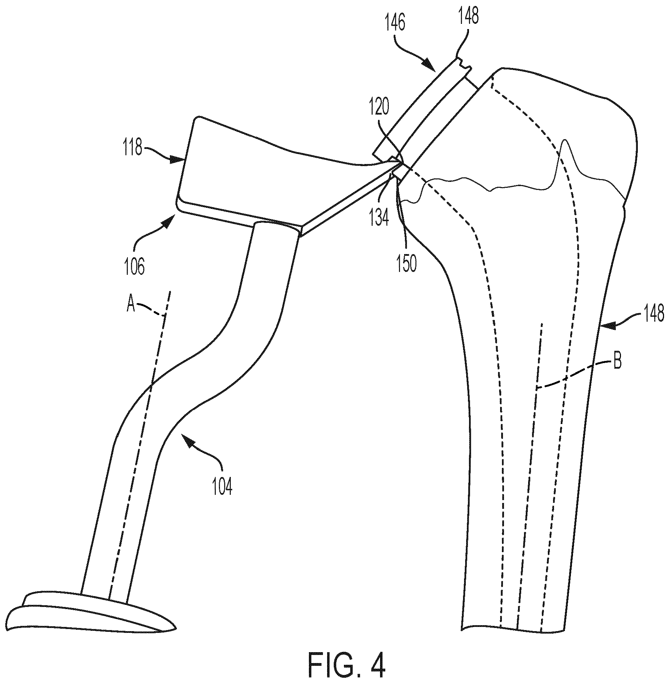

The tip 120 defines a most distal end of the medical implant extractor 100. Further, the tip 120 extends past a most lateral end 132 of the handle a distance D₁ of about 0 to 150 mm. As best illustrated in FIG. 2, the tip 120 includes a recess 134 about its midportion. The recess 134 is configured to receivingly engage with a rib 150 of a collared shoulder stem implant 146 (FIG. 4). For example, the recess can be a concave recess or any other suitable recess or notch sufficient to receive a rib-like feature. In addition, the tip 120 is laterally spaced from the first axial segment a distance "D" that is greater than the distance the second axial segment 112 is laterally spaced from the first axial segment 108.

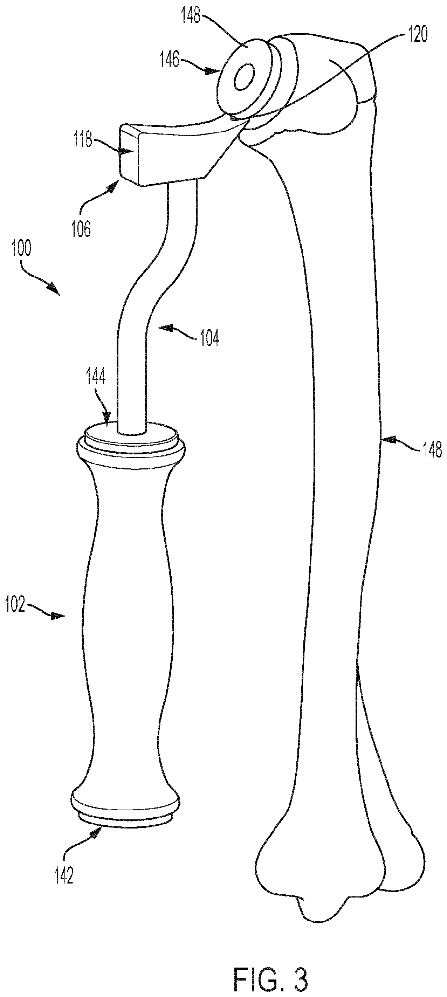

Referring to FIGS. 3 and 4, the medical implant extractor 100 is shown in an implant extraction position. In the illustrated example, the medical implant extractor is shown as it would be positioned to extract a collared shoulder stem implant 146 from an upper region of a humerus bone 148. The medical implant extractor, however, is not so limited in application. As noted above, the medical implant extractor can be used to extract, without limitation, a glenosphere implant, a glenoid implant, a knee implant, a hip implant, an elbow implant or other implant from bone. As shown in FIGS. 3 and 4, the tip 120 can be inserted behind a collar 148 of the implant 146. The tip 120 is designed so that a longitudinal axis "A" of the shank 104 is substantially parallel to a longitudinal axis "B" of the shoulder stem implant 146 to facilitate implant removal in-line with the stem of the implant, thereby minimizing bone loss.

As noted above, the implant may be provided with at least one outwardly projecting rib 150. The receiving engagement of the recess 134 with the rib 150 serves to maintain contact of the tip 120 with the implant 146 during extraction, especially when either the strike plate 118 or second strike plate 142 is struck by an unillustrated striking tool such as a mallet, hammer or the like.

With the tip 120 positioned as in FIGS. 3 and 4, the user may strike either or both of the strike plate 118 and the strike plate 142 with a striking tool to dislodge the implant 146 from the bone 148. The strike plate 118 functions to drive the tip 120 under the implant 146 and into bone and surrounding bodily tissue. The strike plate 142 is primarily used for implant extraction once the tip 120 is properly positioned behind the collar 148 and into surrounding bone and bodily tissue. If the implant does not become fully dislodged, the user can move the tip 120 to a different position behind the implant collar 148 and again strike either or both of the strike plate 118 and the strike plate 142 with a striking tool. The user repeats the foregoing process until the implant 146 is fully dislodged. If during the extraction process the tip 120 slides from the implant 146 and becomes embedded in bone or other bodily tissue, the user may strike the third strike plate 144 until the tip is dislodged from the bodily tissue.

It will be appreciated by those skilled in the art that changes could be made to the examples described above. It is to be understood, therefore, that this disclosure is not limited to the particular examples disclosed, but it is intended to cover modifications within the scope of the subject invention as defined by the appended claims.

## Claims

1. A medical implant extractor (100) comprising a handle (102), a shank (104) and a head (106), wherein:
the shank (104) extends from the handle (102), the shank (104) including a first axial segment (108) having a first longitudinal axis (110) and a second axial segment (112) having a second longitudinal axis (114) spaced from the first longitudinal axis (110), the first axial segment (108) is spaced axially from the second axial segment (112) about 0 to 200 mm;
the head (106) is connected to a distal end of the second axial segment (112), the head (106) including:
a main body (116), and
a strike plate (118); **characterised in that** the strike plate (118) comprises a face orientated substantially parallel to the longitudinal axis of one of the first and second longitudinal axes (110, 114) of the first and second axial segments (108, 112), and that the medical implant extractor (100) further comprises a tip (120) extending distally and outwardly from the main body (116), wherein
the tip (120) is laterally spaced from the first axial segment (108) a distance greater than the second axial segment (112) is laterally spaced from the first axial segment (108).

2. The medical implant extractor (100) of claim 1, wherein the shank includes a first curved segment (122) and a second curved segment (124).

3. The medical implant extractor (100) of claims 1-2, wherein the second axial segment (112) is spaced axially from the first axial segment (108).

4. The medical implant extractor (100) of claims 1-3, wherein the first axial segment (108) is spaced laterally from the second axial segment (112) about 0 to 150 mm.

5. The medical implant extractor (100) of claims 1-4, wherein the second axial segment (112) is laterally spaced from the first axial segment (108).

6. The medical implant extractor (100) of claims 1-5, wherein the tip (120) defines a most distal end of the medical implant extractor (100).

7. The medical implant extractor (100) of claims 1-6, wherein the tip (120) includes a recess (134) about its midportion.

8. The medical implant extractor (100) of claims 1-7, wherein the tip (120) extends past a most lateral end (132) of the handle about 0 to 150 mm.

9. The medical implant extractor (100) of claims 1-8, wherein the head (106) includes a tapered end (126) defining the tip (120).

10. The medical implant extractor (100) of claims 1-9, wherein the head (106) includes a distally facing end (128) that is substantially curved, or wherein the head (106) includes a distally facing end (128) that includes a plurality of planar segments (130a, 130b, 130c).

11. The medical implant extractor (100) of claims 1-10, wherein the strike plate (118) is positioned between planes defined by the lateral most ends of the handle (102).

12. The medical implant extractor (100) of claims 1-11, further comprising a second strike plate (142) about a proximal facing end of the handle (102).

13. The medical implant extractor (100) of claim 12, further comprising a third strike plate (144) about a distally facing end of the handle (102).

## Patentansprüche

1. Extraktor für medizinische Implantate (100), umfassend einen Griff (102), einen Schaft (104) und einen Kopf (106), wobei:
sich der Schaft (104) von dem Griff (102) aus erstreckt, wobei der Schaft (104) einen ersten axialen Abschnitt (108) mit einer ersten Längsachse (110) und einen zweiten axialen Abschnitt (112) mit einer zweiten Längsachse (114) aufweist, die von der ersten Längsachse (110) beabstandet ist, wobei der erste axiale Abschnitt (108) um etwa 0 bis 200 mm axial von dem zweiten axialen Abschnitt (112) beabstandet ist;
der Kopf (106) mit einem distalen Ende des zweiten axialen Abschnitts (112) verbunden ist, wobei der Kopf (106) Folgendes aufweist:
einen Hauptkörper (116) und
eine Schlagplatte (118); **dadurch gekennzeichnet, dass** die Schlagplatte (118) eine Fläche umfasst, die im Wesentlichen parallel zu der Längsachse einer der ersten und zweiten Längsachsen (110, 114) der ersten und zweiten axialen Abschnitte (108, 112) ausgerichtet ist, und dass der Extraktor für medizinische Implantate (100) ferner Folgendes umfasst:
eine Spitze (120), die sich distal und nach außen von dem Hauptkörper (116) erstreckt, wobei
die Spitze (120) um einen größeren Abstand lateral von dem ersten axialen Abschnitt (108) beabstandet ist, als der zweite axiale Abschnitt (112) lateral von dem ersten axialen Abschnitt (108) beabstandet ist.

2. Extraktor für medizinische Implantate (100) nach Anspruch 1, wobei der Schaft einen ersten gekrümmten Abschnitt (122) und einen zweiten gekrümmten Abschnitt (124) aufweist.

3. Extraktor für medizinische Implantate (100) nach den Ansprüchen 1 bis 2, wobei der zweite axiale Abschnitt (112) axial von dem ersten axialen Abschnitt (108) beabstandet ist.

4. Extraktor für medizinische Implantate (100) nach den Ansprüchen 1 bis 3, wobei der erste axiale Abschnitt (108) um etwa 0 bis 150 mm lateral von dem zweiten axialen Abschnitt (112) beabstandet ist.

5. Extraktor für medizinische Implantate (100) nach den Ansprüchen 1 bis 4, wobei der zweite axiale Abschnitt (112) lateral von dem ersten axialen Abschnitt (108) beabstandet ist.

6. Extraktor für medizinische Implantate (100) nach den Ansprüchen 1 bis 5, wobei die Spitze (120) ein am weitesten distal gelegenes Ende des Extraktors für medizinische Implantate (100) definiert.

7. Extraktor für medizinische Implantate (100) nach den Ansprüchen 1 bis 6, wobei die Spitze (120) im Bereich ihres Mittelteils eine Aussparung (134) aufweist.

8. Extraktor für medizinische Implantate (100) nach den Ansprüchen 1 bis 7, wobei sich die Spitze (120) um etwa 0 bis 150 mm über ein am weitesten lateral gelegenes Ende (132) des Griffs hinaus erstreckt.

9. Extraktor für medizinische Implantate (100) nach den Ansprüchen 1 bis 8, wobei der Kopf (106) ein sich verjüngendes Ende (126) aufweist, das die Spitze (120) definiert.

10. Extraktor für medizinische Implantate (100) nach den Ansprüchen 1 bis 9, wobei der Kopf (106) ein distal weisendes Ende (128) aufweist, das im Wesentlichen gekrümmt ist, oder wobei der Kopf (106) ein distal weisendes Ende (128) aufweist, das eine Mehrzahl von ebenen Abschnitten (130a, 130b, 130c) aufweist.

11. Extraktor für medizinische Implantate (100) nach den Ansprüchen 1 bis 10, wobei die Schlagplatte (118) zwischen Ebenen angeordnet ist, die durch die am weitesten lateral gelegenen Enden des Griffs (102) definiert sind.

12. Extraktor für medizinische Implantate (100) nach den Ansprüchen 1 bis 11, ferner umfassend eine zweite Schlagplatte (142) im Bereich eines proximal weisenden Endes des Griffs (102).

13. Extraktor für medizinische Implantate (100) nach Anspruch 12, ferner umfassend eine dritte Schlagplatte (144) im Bereich eines distal weisenden Endes des Griffs (102).

## Revendications

1. Extracteur d'implant médical (100) comprenant une poignée (102), une tige (104) et une tête (106), dans lequel :
la tige (104) s'étend à partir de la poignée (102), la tige (104) comportant un premier segment axial (108) ayant un premier axe longitudinal (110) et un second segment axial (112) ayant un second axe longitudinal (114) espacé du premier axe longitudinal (110), le premier segment axial (108) est espacé axialement du second segment axial (112) d'environ 0 à 200 mm ;
la tête (106) est reliée à une extrémité distale du second segment axial (112), la tête (106) comportant :
un corps principal (116), et
une plaque de frappe (118) ; **caractérisé en ce que** la plaque de frappe (118) comprend une face orientée sensiblement parallèlement à l'axe longitudinal de l'un des premier et second axes longitudinaux (110, 114) des premier et second segments axiaux (108, 112), et **en ce que** l'extracteur d'implant médical (100) comprend en outre
une pointe (120) s'étendant distalement et vers l'extérieur à partir du corps principal (116), dans lequel
la pointe (120) est espacée latéralement du premier segment axial (108) d'une distance supérieure à celle dont le second segment axial (112) est espacé latéralement du premier segment axial (108).

2. Extracteur d'implant médical (100) selon la revendication 1, dans lequel la tige comporte un premier segment incurvé (122) et un second segment incurvé (124).

3. Extracteur d'implant médical (100) selon les revendications 1 à 2, dans lequel le second segment axial (112) est espacé axialement du premier segment axial (108).

4. Extracteur d'implant médical (100) selon les revendications 1 à 3, dans lequel le premier segment axial (108) est espacé latéralement du second segment axial (112) d'environ 0 à 150 mm.

5. Extracteur d'implant médical (100) selon les revendications 1 à 4, dans lequel le second segment axial (112) est espacé latéralement du premier segment axial (108).

6. Extracteur d'implant médical (100) selon les revendications 1 à 5, dans lequel la pointe (120) définit une extrémité la plus distale de l'extracteur d'implant médical (100).

7. Extracteur d'implant médical (100) selon les revendications 1 à 6, dans lequel la pointe (120) comporte un évidement (134) au niveau de sa partie médiane.

8. Extracteur d'implant médical (100) selon les revendications 1 à 7, dans lequel la pointe (120) s'étend au-delà d'une extrémité la plus latérale (132) de la poignée d'environ 0 à 150 mm.

9. Extracteur d'implant médical (100) selon les revendications 1 à 8, dans lequel la tête (106) comporte une extrémité effilée (126) définissant la pointe (120).

10. Extracteur d'implant médical (100) selon les revendications 1 à 9, dans lequel la tête (106) comporte une extrémité orientée distalement (128) qui est sensiblement incurvée, ou dans lequel la tête (106) comporte une extrémité orientée distalement (128) qui comporte une pluralité de segments plans (130a, 130b, 130c).

11. Extracteur d'implant médical (100) selon les revendications 1 à 10, dans lequel la plaque de frappe (118) est positionnée entre des plans définis par les extrémités les plus latérales de la poignée (102).

12. Extracteur d'implant médical (100) selon les revendications 1 à 11, comprenant en outre une deuxième plaque de frappe (142) au niveau d'une extrémité orientée proximalement de la poignée (102).

13. Extracteur d'implant médical (100) selon la revendication 12, comprenant en outre une troisième plaque de frappe (144) au niveau d'une extrémité orientée distalement de la poignée (102).
